# EUROPEAN PATENT SPECIFICATION

(11) **EP 3 749 230 B1**
(45) Date of publication and mention of the grant of the patent: **15.03.2023**
(21) Application number: 19721416.6
(22) Date of filing: 04.02.2019
(51) Int. Cl.: A61B 17/32, A61M 3/02, A61B 17/00, A61B 17/16, A61B 90/00, A61M 39/10, A61M 25/09

(54) **APPARATUS AND METHOD FOR SECURING AN ELONGATE MEMBER TO A MEDICAL INSTRUMENT**
VORRICHTUNG UND VERFAHREN ZUR BEFESTIGUNG EINES LÄNGLICHEN ELEMENTS AN EINEM MEDIZINISCHEN INSTRUMENT
APPAREIL ET PROCÉDÉ DE FIXATION D'UN ÉLÉMENT ALLONGÉ À UN INSTRUMENT MÉDICAL

(30) Priority: 05.02.2018 US 201862626440 P
(43) Date of publication of application: 16.12.2020
(73) Proprietor: Stryker European Operations Holdings LLC, Kalamazoo, MI 49002 (US)
(72) Inventor: CUSHEN, Patrick, Eoin, Cork P25 AX88 (IE); WHITFORD, Alan, Peter, Drogheda, Co. Louth A92 W582 (IE)
(74) Representative: Röthinger, Rainer
(86) International application number: PCT/US2019/016510
(87) International publication number: WO 2019/152924

(56) References cited:
- US-A1- 2005 107 786
- US-A1- 2005 234 297
- US-A1- 2010 298 778
- US-A1- 2016 022 277

## Description

### BACKGROUND

Conventional medical procedures routinely involve the use of surgical tools and/or medical instruments to assist medical professionals in approaching, viewing, manipulation, or otherwise effecting treatment at localized surgical sites. In certain applications of medical instruments, such as those that involve the use of high-speed drills, rotating burs, open-window shavers, and the like, necessarily results in the accumulation of heat and/or debris at the surgical site. Here, surgical systems may employ irrigation systems in combination with a medical instrument. The irrigation system typically includes a fluid source and a flexible line or tube coupled to the medical instrument and configured to supply fluid from the fluid source. Therefore, there remains a need in the art for an attachment device for coupling an elongate member or tube to a medical instrument configured to overcome the various disadvantages of conventional irrigation tube coupling mechanisms.

A securement device, system, and method for use with a medical article is known from document US 2010/0298778 A1. The securement device, system, or method may include a body that has a top surface and a bottom surface. The bottom surface has an adhesive compound thereon. A resilient retainer formed from a soft, tacky elastomeric gel or foam is supported by the bottom surface of the body. The resilient retainer receives and secures a medical device. The medical device is secured to the skin of a patient upon affixing the bottom surface to the patient via the adhesive compound.

Devices and methods for detachably engaging an insertion section of an endoscope and selectively articulating an endoscopic surgical access channel are provided in document US 2005/0234297 A1. A device comprises an articulating main body having a stationary first body, an articulatable second body, and coupler having a mounting member and an articulation link member configured to articulatively couple the second body to the first body. The stationary first body has a distal end, a proximal end, and a first longitudinal axis. The articulatable second body has a proximal opening and a distal opening defining an accessory channel member passageway, the distal opening having a second longitudinal axis oriented toward a space exterior to the first body distal end, wherein the second body longitudinal axis is capable of articulating relative to the first body longitudinal axis. The devices and methods of document US 2005/0234297 A1 provide control over the position and/or orientation of a diagnostic, monitoring, scope, sewing device, cutting device, suturing device, forceps, grabbing device, instrument, or other tool within the visual field beyond the distal opening of the endoscope.

Bipolar surgical forceps having argon plasma coagulation capability are shown in document US 2005/0107786 A1. The surgical forceps include in their body a channel for receiving a flexible tube having a wire within it. The flexible tubing and wire within it are connected to a generator that provides electrical energy to the wire and argon or other inert gas to the tube. The flexible tubing travels a portion of the length of the forceps and ends with a coaxial connector in the proximity of the points of the scissors. An argon plasma coagulation sleeve is placed on the point of one or both limbs of the forceps and is connected to the flexible tubing and wire through the coaxial connector.

### SUMMARY

The present disclosure relates generally to an apparatus and method of removably securing an elongate member, such as a flexible line or tube, to a medical instrument. The apparatus comprises an attachment device that may be configured to secure an elongate member, which may include a lumen, to a medical instrument to facilitate irrigation.

An attachment device for securing an elongate member to a medical instrument is defined in claim 1.

The attachment device comprises a liner, a base, and an adhesive. The base comprises a first base aperture and a second base aperture. The adhesive is disposed at least partially between the base and the liner. The liner is positioned such that the liner is at least partially between the first base aperture and the second base aperture, and is adjacent to the adhesive. In addition or alternatively, the liner is configured to cover a portion of the adhesive disposed on the base between the first base aperture and a first outer edge of the attachment device, and between the second base aperture and a second outer edge of the attachment device. The liner is further configured to allow the elongate member to move axially along an axis defined by said first base aperture and said second base aperture when the attachment device is attached to the medical instrument.

An attachment device assembly for securing an elongate member to a medical instrument is defined in claim 6. The attachment device assembly for securing an elongate member to a medical instrument comprises the attachment device and an indicator tab removably coupled to the attachment device, wherein the indicator tab comprises indicia representative of a method of attaching the attachment device to the medical instrument.

An attachment device assembly for use with a medical instrument to facilitate irrigation is defined in claim 8. The attachment device assembly comprises the attachment device and the elongate member, with the elongate member threaded through the first base aperture and the second base aperture, and adapted to provide irrigation.

An irrigation system as defined in claim 12 comprises a medical instrument configured to cut tissue, said medical instrument having a body configured to be held by a user. The irrigation system further comprises an elongate member and the attachment device. The coupling of the elongate member to the medical instrument by the attachment device is removable. The elongate member is threaded through the first base aperture and the second base aperture such that a portion of the elongate member is adjacent to the body of the medical instrument.

A method of coupling an elongate member to a medical instrument as defined in claim 14 comprises the step of providing the attachment device. The method further comprises the step of threading an elongate member through the first base aperture and the second base aperture such that the elongate member is axially aligned with the liner. The method also comprises the step of adhering the attachment device to the medical instrument, wherein an exposed portion of the adhesive removably couples the attachment device to the medical instrument such that the elongate member is at least partially disposed between the attachment device and the medical instrument.

These and other embodiments, features, and advantages of the present disclosure will be apparent to those skilled in the art. The present disclosure is not to be limited to or by these embodiments, features, and advantages.

### BRIEF DESCRIPTION OF THE DRAWINGS

Referring now to the drawings, exemplary illustrations are shown in detail. Although the drawings represent schematic embodiments, the drawings are not necessarily to scale and certain features may be exaggerated to better illustrate and explain an innovative aspect of an illustrative embodiment. Further, the exemplary illustrations described herein are not intended to be exhaustive or otherwise limiting or restricting to the precise form and configuration shown in the drawings and disclosed in the following detailed description.

Advantages of the present disclosure will be readily appreciated as the same becomes better understood by reference to the following detailed description when considered in connection with the accompanying drawings.
Figure 1 is a top view of an attachment device for securing an elongate member to a medical instrument.
Figure 2 is a perspective view of the attachment device of Figure 1.
Figure 3 is a sectional view of the attachment device of Figure 1, illustrating an example configuration of the various elements of the attachment device.
Figure 4A is a bottom view of the attachment device of Figure 1, illustrating a first embodiment of a liner.
Figure 4B is a rotated sectional view of the attachment device of Figure 4A including the first embodiment of the liner.
Figure 5A is a bottom view of the attachment device of Figure 1, illustrating a second embodiment of a liner.
Figure 5B is a rotated sectional view of the attachment device of Figure 5A including the second embodiment of the liner.
Figure 6A is a bottom view of the attachment device of Figure 1, illustrating a third embodiment of a liner.
Figure 6B is a rotated sectional view of the attachment device of Figure 6A including the third embodiment of the liner.
Figure 7 is a top view of an attachment device assembly including an indicator tab and the attachment device of Figure 1.
Figure 8 is a partially exploded view of the attachment device assembly of Figure 7.
Figure 9 is a top view of an indicator tab including an example illustration of indicia on the indicator tab representing a method of securing an elongate member to a medical instrument using the attachment device.
Figure 10 is a perspective view of a partially exploded and fully assembled attachment device assembly including an elongate member and an irrigation sleeve.
Figure 11 is a top and bottom view of the assembled attachment device assembly of Figure 10 including an elongate member and an irrigation sleeve.
Figure 12A is a perspective view of a method of securing an elongate member to a medical instrument using the attachment device of Figure 1.
Figure 12B is a perspective view of the method of Figure 12A of securing an elongate member to a medical instrument using the attachment device of Figure 1.
Figure 12C is a perspective view of the method of Figure 12A of securing an elongate member to a medical instrument using the attachment device of Figure 1.

### DETAILED DESCRIPTION

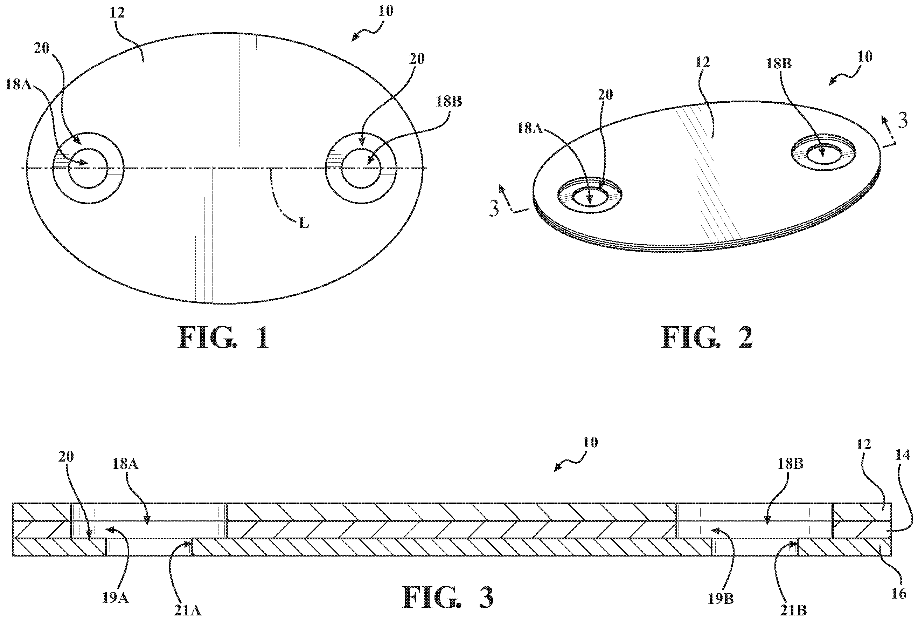

As medical professionals strive for reducing the size of the incisions and the amount of recovery time required following invasive medical procedures, the size of medical instruments used in various medical procedures have become smaller. Many of the medical instruments utilized in performing the various medical procedures may include the use of a cutting accessory, such as high-speed drills, rotating burs, open-window shavers, and the like. An exemplary medical instrument is disclosed in U.S. Patent No. 5,941,891, granted on Aug. 24, 1999. Many of these cutting accessories may require the use of irrigation or suction to reduce heat and/or remove debris at the surgical site. Similarly, irrigation may be utilized to lubricate a cutting accessory. Generally, an elongate member, such as those including a lumen, may be utilized to direct fluid from an irrigation source toward the surgical site and/or the cutting accessory. The elongate member may be disposable as part of the sterilization process, resulting in the elongate member only temporarily being attached to the medical instrument. The introduction of additional elements, such as the elongate member for providing irrigation, can result in increasing the size, profile, and/or general bulkiness of the medical instrument. A loose elongate member extending from the irrigation system to the cutting accessory may obstruct the medical professional's view and/or distract the medical professional during operation of the medical instrument during the medical procedure. The positioning of the elongate member relative to the medical instrument may similarly effect the ergonomics of the medical instrument, inhibiting the medical professional's ability to manipulate the medical instrument. Therefore, the medical professional may utilize an attachment device and/or assembly for securing an elongate member to a medical instrument, such as the attachment device 10 illustrated in Figures 1 and 2.

Referring to Figures 1, 2, and 3, an example configuration of an attachment device 10 is illustrated. Accordingly, the attachment device 10 may comprise a base 12, adhesive 14, and a liner 16, wherein the attachment device 10 is configured for attachment to a medical instrument 48. The attachment device 10 may be configured as a laminate, wherein each of the base 12, adhesive 14, and liner 16 makeup the various layers of the attachment device 10 as shown in Figure 3, and may by positioned directly adjacent one another. For example, referring to Figures 2 and 3, the attachment device 10 comprises a base 12, an adhesive 14, and a liner 16 that have been layered on top of one another. Alternatively, the base 12, adhesive 14, and liner 16 may be configured as individual elements that may be assembled using the adhesive 14 to interconnect the base 12 and the liner 16.

The base 12 may comprise a material that is flexible and tear-resistant. For example, the base may comprise a plastic or composite material. The base 12 may similarly be resistant to degradation or erosion when utilized in a medical setting, such as a hospital or operating room. For example, the base 12 may be cut in various shapes, such as a generally elliptical shape, as illustrated in Figures 1, 2, and 3 to define one or more apertures (18A, 18B) in a complementary shape, as described below. The generally elliptical shape may provide the necessary structural properties while also providing a limited profile when coupling the attachment device 10 to a medical instrument having a generally convex surface. While not shown in the figures, it is contemplated that the base 12 may be cut in different shapes and/or configurations based on the application. For example, the base 12 may be cut in a square, rectangular, or similar polygonal shape. This may provide alternative structural properties and/or advantages when coupling the attachment device 10 to a generally flat surface. However, the examples described above are not intended to be limiting. For example, it is contemplated that the polygonal-shaped base may be coupled to a convex surface and the generally circular-shaped base 12 may be coupled to a flat surface.

The base 12 may be at least partially translucent and treated for printing, such that text and/or graphics may be printed on a portion of the base 12. For example, the base 12 may include printed identifying marks or graphics, as well as indicia representative of instructions for using the attachment device 10. The base 12 may also be markable, i.e., include a writing surface, such that a medical professional may write a date of use or installation on the base 12 using a typical writing utensil, such as a pen, marker, or pencil.

The adhesive 14 may be generally disposed on at least one surface of the base 12 such that a portion of the adhesive 14 may be positioned between the base 12 and the liner 16. For example, the adhesive 14 may be applied to or cover the entire area of at least one surface, such as the bottom, of the base 12, and the liner 16 may be configured to cover a defined area of the adhesive 14, leaving a portion of the adhesive disposed on the base 12 exposed. The exposed portion of the adhesive 14 (not covered by the liner 16) may be configured to removably couple the attachment device 10 to a medical instrument 48 (discussed in detail below). In other words, the liner remains on the attachment device 10 after the attachment device is secured to the medical instrument 48.

The adhesive 14 may comprise a chemical compound, such as a paste, silicone, glue, rubber cement, or similar adhesive substance configured to adhere the base 12 to the liner 16 and/or the medical instrument 48. The adhesive 14 may similarly be configured as an adhesive tape, such as double-sided tape with an adhesive on both sides to allow for adhesion to both the base 12 and the liner 16. For example, the adhesive 14 may be a medical grade adhesive manufactured by 3M^{™}, such as 3M^{™} 9865 Polyethylene Single Sided Medical Tape. The adhesive 14 may be configured such that it is suitable for use in wet and/or unclean environments and is safe for attachment to human skin.

The liner 16 is configured to be coupled to the adhesive, such that the liner 16 may be positioned adjacent, such as directly adjacent, to the adhesive 14 opposite the base 12. The liner 16 may be configured to be disposed on the adhesive 14, such that the liner 16 covers a portion or defined area of the adhesive 14. The liner 16 may be removably coupled to the adhesive 14 of the attachment device 10. The liner 16 may comprise material that has been treated with a chemical compound that allows for easy removal of the liner 16 from the adhesive 14. For example, the liner 16 may comprise a wax or Teflon^{™} coating. The liner 16 may be thought of as being similar to the backing of a sticker, wherein the liner 16 may remain coupled to the adhesive 14, but can be easily removed or separated from the adhesive 14. However, at least of the portion of the liner 16 may remain on the attachment device when the attachment device is secured to the medical instrument.

As illustrated in Figures 1, 2, and 3, the attachment device 10 may comprise a first base aperture 18A and a second base aperture 18B in the base 12. While the figures illustrate a configuration of the attachment device 10 comprising the first base aperture 18A and the second base aperture 18B, it should be understood that it is contemplated that the attachment device 10 may be configured with a single base aperture, or three or more base apertures. Depending on the location, shape, and/or number of base apertures 18A, 18B, the position and configuration of the adhesive 14 and/or liner 16 may be altered, including apertures thereof, which will be discussed in detail below.

The first base aperture 18A and the second base aperture 18B may also be configured to define a longitudinal axis L, as illustrated in Figure 1. For example, the longitudinal axis L bisects the first base aperture 18A and the second base aperture 18B. In one or more of the configurations of the attachment device 10 described below, the liner 16 may be configured to be substantially aligned with the longitudinal axis L defined by the first base aperture 18A and the second base aperture 18B. In one aspect, aligned is meant to refer to a longitudinal axis of the liner 16 being parallel to the longitudinal axis L. More generally, in some aspects, at least a portion of the liner 16 is disposed on the adhesive 14 between the first base aperture 18A and the second base aperture 18B. In other words, in certain aspects, the liner 16 may be arranged in other fashions relative to the longitudinal axis L and/or the first and second base apertures 18A, 18B.

The attachment device 10 may also comprise an adhesive aperture 19A, 19B in the adhesive 14. The adhesive aperture(s) 19A, 19B may be configured to be aligned, such as coaxial, with the first base aperture 18A and the second base aperture 18B. It is further contemplated that the liner 16 may also comprise a liner aperture(s) 21A, 21B, wherein the liner aperture(s) 21A, 21B may be aligned, such as coaxial, with the first base aperture 18A and the second base aperture 18B and/or the adhesive aperture(s) 19A, 19B. Thus, in one aspect, the base aperture(s) 18A, 18B, the adhesive apertures 19A, 19B, and the liner apertures 21A, 21B may be collectively arranged such that center of each aperture is aligned. However, in other aspects, the base aperture 18A, 18B, the adhesive apertures 19A, 19B, and liner apertures 21A, 21B may be partially coincident such that the elongate member can pass therethrough. The shapes and/or sizes of the base apertures 18A, 18B, adhesive apertures 19A, 19B, or liner apertures 21A, 21B may be the same or may be different. For example, as illustrated in Figures 1 to 3, the size of the base aperture(s) 18A, 18B and/or the adhesive aperture(s) 19A, 19B may be configured to be larger than the liner aperture(s) 21A, 21B in order to prevent transference of the adhesive 14 to an obj ect passing through the base aperture(s) 18A, 18B.

Alternatively, in another configuration of the attachment device 10, the base 12 and/or the may further comprise a recess 20, bevel, chamfer, fillet, or similar transition between opposing surfaces of the base 12 surrounding the base aperture(s) 18A, 18B. The recess 20 surrounding the aperture(s) 18A, 18B may be configured to reduce any stresses or strain on the base 12 at the circumference of the aperture(s) 18A, 18B.

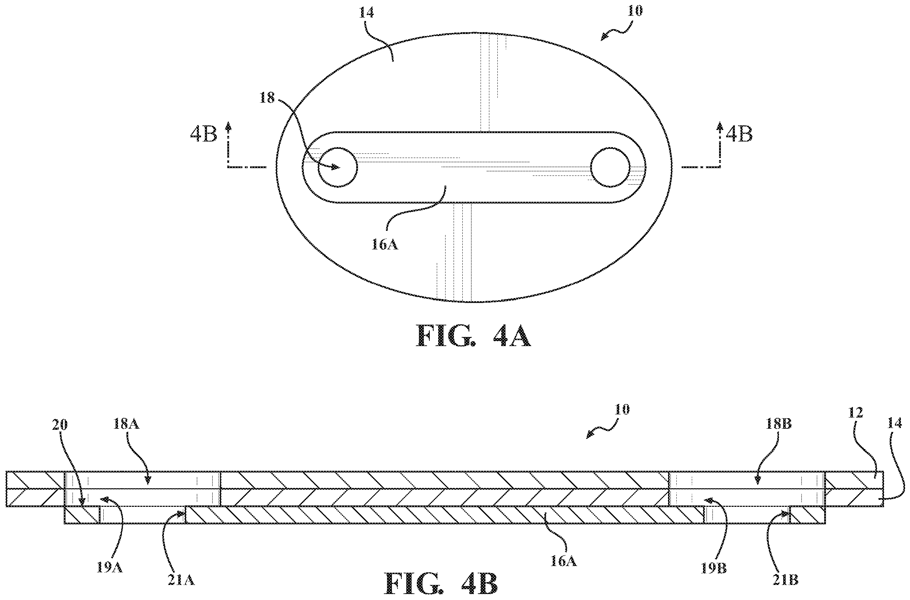

Referring to Figures 4A and 4B, a first exemplary liner 16A configuration is illustrated. In the first exemplary configuration of the liner 16A, the liner 16A is configured such that at least a portion of the liner 16A is removably coupled to the adhesive 14 this disposed on the base 12 between the first and second base apertures 18A, 18B. The liner 16A may also be configured to extend beyond the area of the adhesive 12 disposed on the base 12 between the first and second base apertures 18A, 18B. For example, the liner 16A may be configured to also cover a portion of the adhesive 14 that is disposed on the base 12 surrounding the first and second base apertures 18A, 18B, as illustrated in Figures 4A and 4B, that is not on the base between the first and second apertures 18A, 18B. In this liner 16A configuration, the liner 16A may comprise liner apertures 21A, 21B.

The sectional view illustrated in Figure 4B illustrates an attachment device 10 wherein the base aperture(s) 18A, 18B, the adhesive aperture(s) 19A, 19B each comprise a generally uniform diameter. The liner aperture(s) 21A, 21B may be configured to be smaller relative to the size of the base aperture(s) 18A, 18B and /or the adhesive aperture(s) 19A, 19B. This may prevent the transference of the adhesive 14 to an object passing through the base aperture(s) 18A, 18B. While not shown in the figures, it is contemplated that the size of the liner aperture(s) 21A, 21B of the attachment device 10 may be may be the same size as the base aperture(s) 18A, 18B and /or the adhesive aperture(s) 19A, 19B. For example, the base aperture(s) 18A, 18B, the adhesive aperture(s) 19A, 19B and/or liner aperture(s) 21A, 21B may be configured to comprise a uniform size, such as diameter, through the attachment device 10. It is also contemplated that the first base 18A, first liner 19A, and first adhesive 21A apertures may be sized and/or shaped differently than the second base 18B, second liner 19B, and second adhesive 21B apertures.

The base aperture(s) 18A, 18B, the adhesive aperture(s) 19A, 19B, and the liner aperture(s) 21A, 21B may be generally aligned, such coaxial, when the base 12, adhesive 14, and/or liner 16 are layered or stacked on top of one another, such that the base aperture(s) 18A, 18B, the adhesive aperture(s) 19A, 19B, and the liner aperture(s) 21A, 21B form a continuous aperture through the attachment device 10. Alternatively, it is also contemplated that the size, shape, and/or position of each aperture may be different. For example, the center of the base aperture(s) 18A, 18B and/or the adhesive aperture(s) 19A, 19B may be offset from the center of the liner aperture(s) 21A, 21B. In yet another configuration, the shape of the base aperture(s) 18A, 18B and/or the adhesive aperture(s) 19A, 19B may be different from the shape of the liner aperture(s) 21A, 21B. For example, the liner aperture(s) 21A, 21B may be configured as a circle and the base aperture(s) 18A, 18B and/or the adhesive aperture(s) 19A, 19B may be configured as an ellipse. This configuration may assist with preventing transfer of the adhesive 14 to the elongate member 36.

In another configuration, a portion of the adhesive 14 may be removed from the area of the base 12 that surrounds the base aperture(s) 18A, 18B, increasing the size, such as the diameter, of the adhesive aperture(s) 19A, 19B. In other words, a portion of the base 12 surrounding the base aperture(s) 18A, 18B is not in direct contact with the adhesive 14. A potential advantage of increasing the size, shape, and/or position of the base aperture 18A, 18B and/or adhesive aperture(s) 19A, 19B relative to the liner aperture(s) 21A, 21B is to prevent oozing or transference of the adhesive 14 onto the elongate member 36 when threaded through the base apertures 18A, 18B of the attachment device 10.

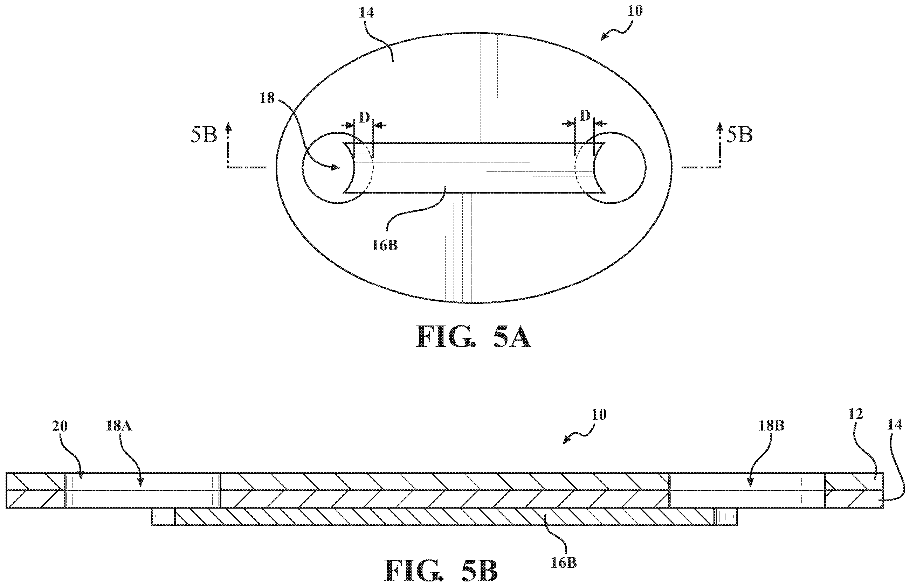

Referring to Figures 5A and 5B, a second exemplary liner 16B configuration is illustrated. In the second exemplary configuration of the liner 16B, the liner 16B is configured such that at least a portion of the liner 16B is coupled to the adhesive 14 that is disposed on the base between the first and second base apertures 18A, 18B. In the second configuration of the liner 16B, the portion of the liner 16B that is coupled to the adhesive 14 may not surround the first and second base apertures 18A, 18B and/or the adhesive aperture(s) 19A, 19B. The liner 16B, as illustrated in Figures 5A and 5B, is configured as a strip, belt, strap, band, or ribbon of material coupled to a portion of the adhesive 14 that is disposed on the base 12 between the first and second base apertures 18A, 18B.

The length and width of the liner 16B may be configured based on the size of the base aperture(s) 18A, 18B, and/or the adhesive aperture(s) 19A, 19B, as well as the dimensions of the elongate member 36. The length and width of the liner 16B may also be configured based on the distance between the base aperture(s) 18A, 18B, and/or the adhesive aperture(s) 19A, 19B. While not required, the width of the liner 16B may typically be at least as large as the size of the base aperture(s) 18A, 18B, and/or the adhesive aperture(s) 19A, 19B. For example, the width of the liner 16B may the same or larger that the diameter of the base aperture(s) 18A, 18B and/or the adhesive aperture(s) 19A, 19B. The length of the liner 16B may also be dependent on the size of the adhesive aperture(s) 19A, 19B. For example, in order to prevent a portion of the adhesive 14 disposed on the base 12 that covers the area between the base aperture(s) 18A, 18B from being exposed, the edges of the liner 16B, the liner 16B may be configured to cover at least the portion of adhesive 14 disposed on the base 12 between the base aperture(s) 18A, 18B. As illustrated in Figures 5A and 5B, the length of the liner 16B may be longer the distance between the edges of the base aperture(s) 18A, 18B and/or the adhesive aperture(s) 19A, 19B, such the liner 16B extends into and coves a portion of the base aperture(s) 18A, 18B and/or the adhesive aperture(s) 19A, 19B. Alternatively, if the adhesive 14 is flush with the base aperture(s) 18A, 18B, then the liner 16B may be similarly configured to extend between the edges of the base aperture(s) 18A, 18B, such that the liner 16B is also flush with the edge(s) of the base aperture(s) 18A, 18B. However, if a portion of the adhesive 14 is removed surrounding the base aperture(s) 18A, 18B, the length of the liner 16B may be shortened, such that the liner 16B will only cover the area on the base 12 that is between the base aperture(s) 18A, 18B that is in contact with the adhesive 14.

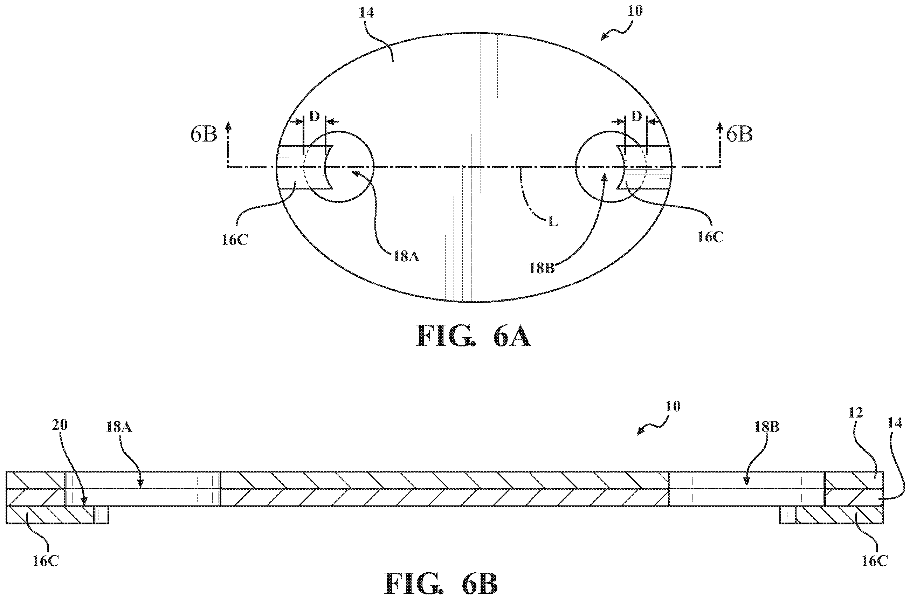

Referring to Figures 6A and 6B, a third exemplary liner 16C configuration is illustrated. Similar to the liner 16A, 16B configurations illustrated in Figures 4A-5B, the third exemplary configuration of the liner 16C may be coupled to the adhesive 14 and may be positioned adjacent the base aperture(s) 18A, 18B. The liner 16C may be couple to a portion of the adhesive 14 such that the liner 16C is substantially parallel, or collinear, with a longitudinal axis L that may bisect the first base aperture 18A and the second base aperture 18B. The liner 16C may be configured to be substantially aligned with the longitudinal axis L defined by the first base aperture 18A and the second base aperture 18B, wherein aligned is meant to refer to a longitudinal axis of the liner 16 being parallel to the longitudinal axis L. As illustrated in Figures 6A and 6B, it is not required that the liner 16C be disposed on a portion of the adhesive 14 between the first and second base apertures 18A, 18B. In the example configuration illustrated in Figures 6A and 6B, the liner 16C may comprise multiple pieces disconnected from one another and positioned adjacent to the base aperture(s) 18A, 18B, wherein the pieces of the liner 16C veer out from the base aperture(s) 18A, 18B relative to the center of the attachment device 10. Alternatively, the liner 16C may be disposed on a portion of the adhesive 14 that is disposed on the base 12 that is not between the first and second base apertures 18A, 18B, but is adjacent to the first and/or second base apertures 18A, 18B.

The size and/or position of the liner 16C may be based, at least in part, on the size and/or position of the base aperture(s) 18A, 18B and/or the adhesive aperture(s) 19A, 19B, as well as the dimensions of the elongate member 36. The length and width of the liner 16C may also be configured based on the distance between the base aperture(s) 18A, 18B, and/or the adhesive aperture(s) 19A, 19B, and the outer edge of the attachment device 10. While not required, the width of the liner 16C may typically be at least as large as the size of the base aperture(s) 18A, 18B, and/or the adhesive aperture(s) 19A, 19B. For example, the width of the liner 16C may the same or larger that the diameter of the base aperture(s) 18A, 18B and/or the adhesive aperture(s) 19A, 19B. The length of the liner 16C may also be dependent on the size of the adhesive aperture(s) 19A, 19B. For example, in order to prevent a portion of the adhesive 14 disposed on the base 12 that covers the area between the base aperture(s) 18A, 18B and the outer edge(s) of the attachment device 10 from being exposed, the edges of the liner 16C, the liner 16C may be configured to cover at least the portion of adhesive 14 disposed on the base 12 between the base aperture(s) 18A, 18B and the outer edge(s) of the attachment device 10. As illustrated in Figures 6A and 6B, the length of the liner 16C may be longer the distance between the edges of the base aperture(s) 18A, 18B and the outer edges of the attachment device 10, such the liner 16C extends into and coves a portion of the base aperture(s) 18A, 18B and/or the adhesive aperture(s) 19A, 19B. Alternatively, if the adhesive 14 is flush with the base aperture(s) 18A, 18B, then the liner 16C may be similarly configured to extend between the edges of the base aperture(s) 18A, 18B, such that the liner 16C is also flush with the edge(s) of the base aperture(s) 18A, 18B. However, if a portion of the adhesive 14 is removed surrounding the base aperture(s) 18A, 18B, the length of the liner 16C may be shortened, such that the liner 16C will only cover the area on the base 12 that is between the base aperture(s) 18A, 18B and the outer edge(s) of the attachment device 10 that is in contact with the adhesive 14.

While various configurations of the liner 16 have been discussed above, it should be understood that it is also contemplated that the attachment device 10 may be configured without a liner. In an embodiment of the attachment device 10, that does not include the liner, a portion of the adhesive that corresponds to the same general location, position, and/or shape that the liner may be removed from the attachment device 10. For example, the attachment device 10 may be configured to comprise a base 12 including a top surface and a bottom surface. As described above, the attachment device 10 may similarly comprises the first base aperture 18A, and the second base aperture 18B. The adhesive 14 may be at least partially disposed on the bottom surface of the base 12, such that the area of the base 12 base 12 between the first base aperture 18A and the second base aperture 18B is free from adhesive. The attachment device 10 may be configured wherein the area of the base 12 that is free from adhesive may correspond to the areas described above where the adhesive is covered by the liner.

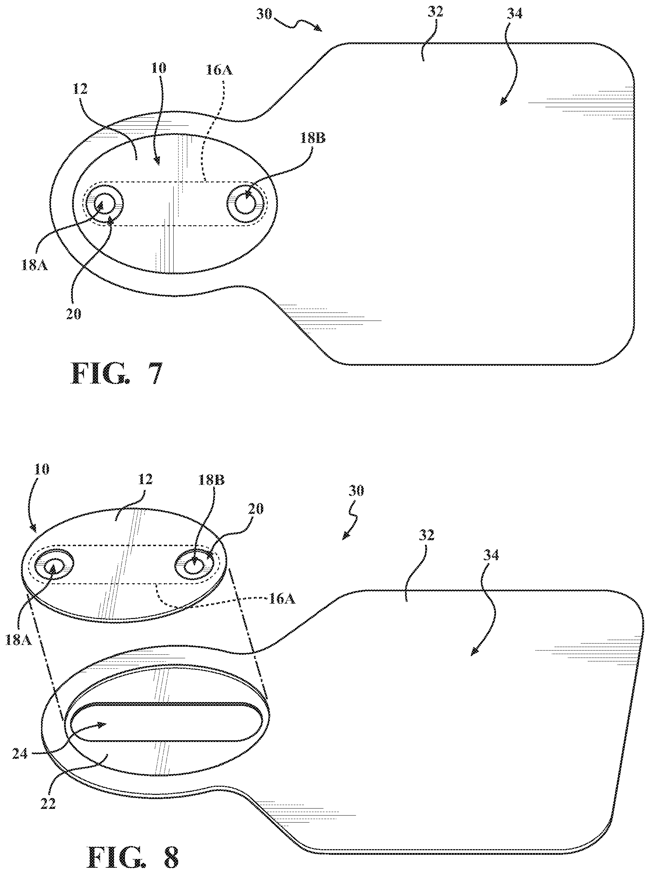

Referring to Figures 7 and 8, an exemplary configuration of an attachment device assembly 30 is illustrated. The attachment device assembly 30 may comprise the attachment device 10 described above and an indicator tab 32. The indicator tab 32 may comprise a second liner 22 configured to removably couple to the adhesive 14 of the attachment device 10 that is not covered by the liner 16. The second liner 22 and the liner 16 may comprise similar or the same materials. The liner 16 and second liner 22 may even be integrally formed from a single piece of material, wherein the material is cut to create the liner 16 and second liner 22. The liner 16 and second liner 22 may be cut such that the liner 16 is configured to remain coupled to the adhesive 14 of the attachment device 10 when the attachment device 10 is removed and/or separated from the indicator tab 32. For example, as illustrated in Figure 8, the second liner 22 comprises a second liner aperture 24 corresponding to the shape of the liner 16 that remains coupled to the attachment device 10. When the second liner 22 is removed by separating the indicator tab 32 from the attachment device 10, a portion of the adhesive 14 that is disposed on the base 12 of the attachment device 10 corresponding to the second liner 22 is exposed. The exposed portion of the adhesive 14 may be configured to secure the attachment device 10 to another object, such as a medical instrument 48.

The second liner 22 may be integrally formed with the indicator tab 32 of the attachment device assembly 30. However, while not illustrated in the figures, it is contemplated that the second liner 22 may be configured as an element of the indicator tab 32. For example, the indicator tab 32 may be constructed of a laminate material or layers similar to the attachment device 10 described above. In this example configuration, the indicator tab 32 may comprise a base indicator layer, an adhesive indicator layer, and the second liner, wherein the second liner is removably coupled to the adhesive indicator layer.

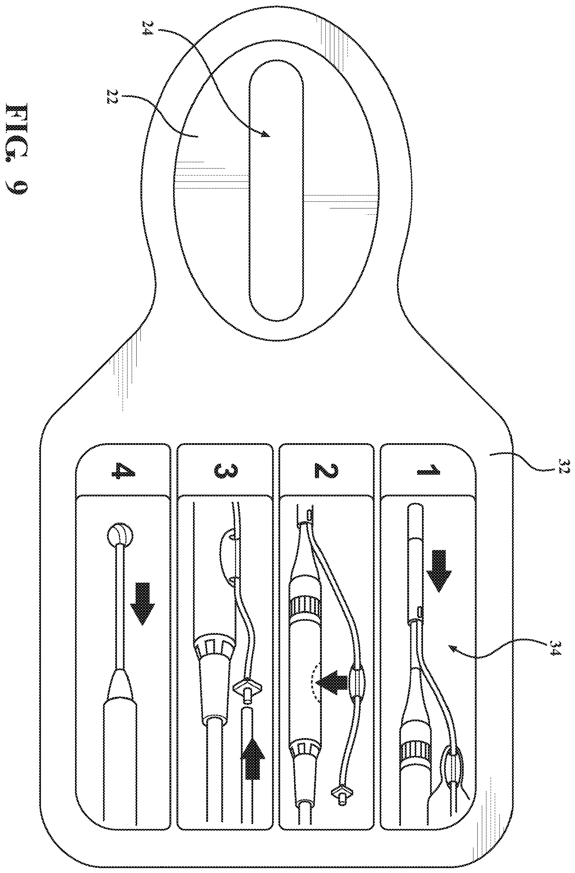

The indicator tab 32 of the attachment device assembly 30 may further comprise an indicia portion 34. The indicia portion 34, similar to the base 12 of the attachment device 10, may be translucent and treated for printing, such that text and/or graphics may be printed on the indicia portion 34. For example, the indicia portion 34 may include printed identifying marks, illustrations, text, or graphics that are representative of instructions for using the attachment device 10. The indicia portion 34 may also be markable, such that a medical professional may write a date of use or installation on the indicia portion 34 using a typical writing utensil, such as a pen, marker, or pencil. Referring to Figure 9, an example illustration of the indicia portion 34 of the indicator tab 32 is shown.

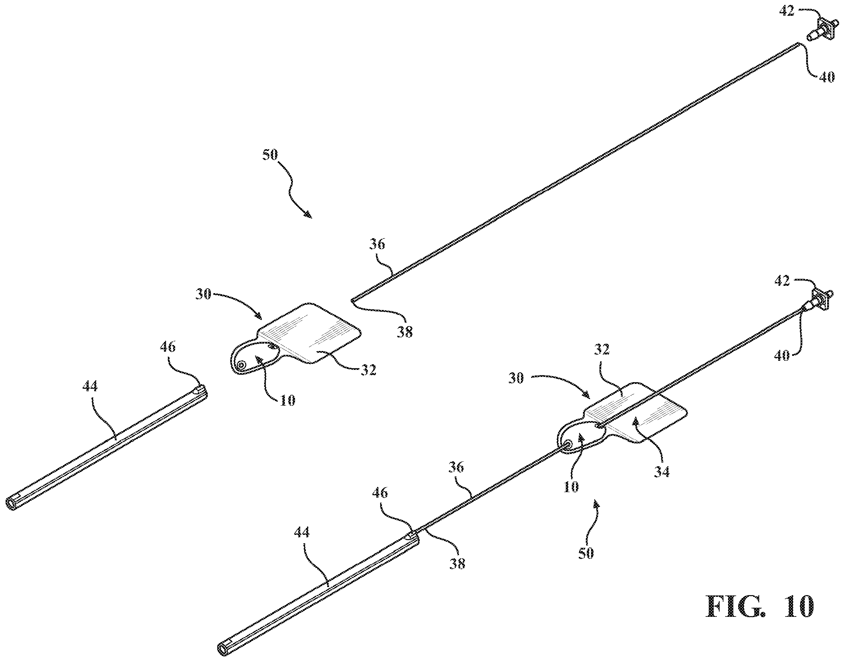

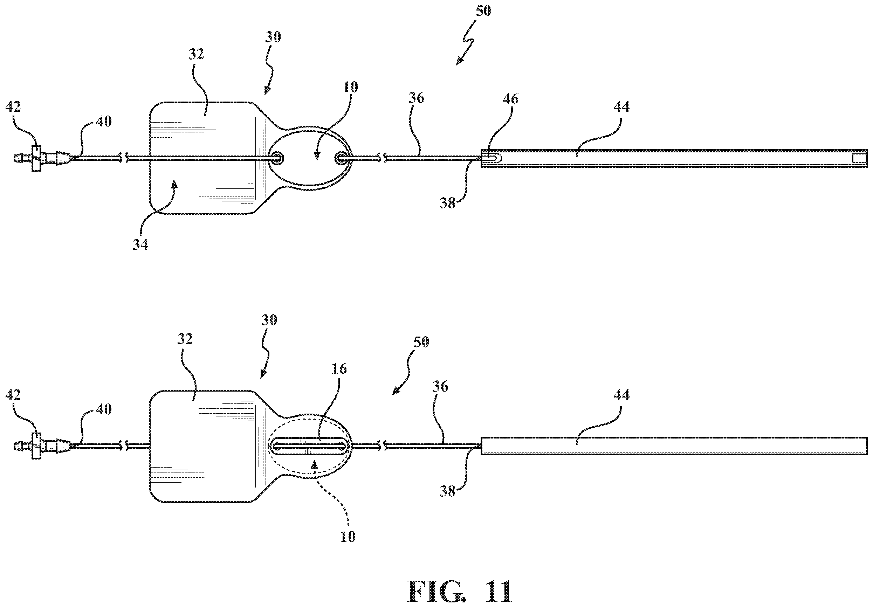

Illustrated in Figures 10 and 11 is an exemplary configuration of an irrigation assembly 50, including an elongate member 36 threaded through the attachment device assembly 30 described above. The elongate member 36 may be constructed from a plastic, a polymer, or similar durable and flexible material. The elongate member 36 may also comprise a lumen configured to create a canal, duct, or passageway that allows for the flow of a fluid. For example, the elongate member may be used to provide fluid to the surgical site, or suction debris away from the surgical site through the elongate member 36. The elongate member 36 may be configured for a navigation tracker of a navigation system, or a control console.

The elongate member may further comprise a distal end 38 and a proximal end 40. The proximal end 38 may be configured to couple to a surgical system, such as an irrigation system (not illustrated in the figures). The proximal end 40 may comprise a proximal coupler 42, wherein the proximal coupler 42 may be configured to couple the proximal end 40 to the surgical system. The proximal coupler 42 may comprise a barbed connector, a luer connector, electrical connector, or similar fitting. It will be appreciated that the proximal coupler 42 may be coupled to the proximal end 40 of the elongate member 36 in a number of different ways, such as via ultraviolet bonding, gluing, cement, epoxy, plastic weld, and the like. It should also be appreciated that the proximal coupler 42 may be omitted, and the elongate member 36 may be coupled directly to the surgical system.

The distal end 38 may be configured to couple to a medical instrument accessory 44, such as an irrigation sleeve. An exemplary irrigation sleeve for use with a medical instrument is disclosed in U.S. Patent Application 16/633,202. The distal end 38 of the elongate member may comprise a barbed connector, a luer connector, or similar fitting configured to couple the elongate member 36 to the medical instrument accessory 44. Alternatively, the medical instrument accessory 44 may comprise an accessory coupler 46 configured to receive the distal end 38 of the elongate member 36. The accessory coupler 46 may be coupled to the distal end 38 of the elongate member 36 in a number of different ways, such as via ultraviolet bonding, gluing, cement, epoxy, plastic weld, and the like. It should also be appreciated that the accessory coupler 46 may be omitted, and the elongate member 36 may be configured to be positioned proximate to the medical instrument accessory 44 and apply fluid directly to the surgical sight.

As described above, the attachment device 10 may comprise a number of base apertures 18A, 18B. The base aperture(s) 18A, 18B may be configured to receive the elongate member 36, which may be threaded through the base aperture(s) 18A, 18B in the attachment device 10. The dimensions and/or thickness of the base aperture(s) 18A, 18B may be configured to reduce any stresses or strain on the base 12 and/or elongate member 36 when the elongate member 36 is threaded through the base aperture(s) 18A, 18B. The diameter of the base aperture(s) 18A, 18B and/or distance between the base aperture(s) 18A, 18B may be configured to create a friction fit for the elongate member 36 when threaded through the base aperture(s) 18A, 18B. This will hold the elongate member 36 in place, unless an additional force is applied to the elongate member 36, such as the medical professional pulling the elongate member 36 in one direction or the other. Alternatively, as discussed above, the base may also comprise a recess 20, wherein the dimensions and/or thickness of the recess 20 surrounding the base aperture(s) 18A, 18B may be configured to reduce any stresses or strain on the base 12 and/or elongate member 36 when the elongate member 36 is threaded through the base aperture(s) 18A, 18B.

As discussed above, there are a number of different liner 16A, 16B, and 16C configurations that may be utilized. However, the liner 16A, 16B, and 16C configuration may dictate the direction and orientation with which the elongate member 36 is threaded through the base aperture(s) 18A, 18B of the attachment device 10. Referring to Figures 10 and 11, the threading configuration of the elongate member 36 with relation to the attachment device 10 would correspond to the liner 16A, 16B illustrated in Figures 4A to 5B, wherein the liner 16A, 16B is positioned adjacent the adhesive 14 and at least partially between the base aperture(s) 18A, 18B. The elongate member 36 is threaded through the base aperture(s) 18A, 18B of the attachment device 10 such that an intermediary portion of the elongate member 36 that is positioned between the base aperture(s) 18A, 18B is adjacent to the liner 16A, 16B. Figure 11 shows the elongate member 36 positioned adjacent to the liner 16 as it extends between the base aperture(s) 18A, 18B. One of the many functions of the liner 16 is to prevent the elongate member 36 from being coupled to the adhesive 14, thus allowing the elongate member 36 to be slid axially when attached to the medical instrument 48.

Alternatively, the threading configuration of the elongate member 36 may be configured to correspond to the liner 16C illustrated in Figures 6A and 6B. This would be the inverse of the elongate member 36 threading illustrated in Figures 10 and 11. In the liner 16C configuration illustrated in Figures 6A and 6B, the liner 16C comprises two separate pieces that are placed adjacent to the base aperture(s) 18A, 18B. However, in this configuration, the liner 16C may not be positioned between the base aperture(s) 18A, 18B. In threading the elongate member 36 through the base aperture(s) 18A, 18B, the elongate member 36 is threaded through the base aperture(s) 18A, 18B of the attachment device 10 such that an intermediary portion of the elongate member 36 that is positioned between the base aperture(s) 18A, 18B is adjacent to the base 12, i.e., on the top of the attachment device 10. The opposing ends of the elongate member 36 are positioned adjacent to the pieces of the liner 16C.

While not illustrated in the figures, it should be appreciated that additional base aperture(s) 18A, 18B and/or liner configurations are contemplated to allow for additional threading configurations. Irrespective of the number of base aperture(s) 18A, 18B and/or pieces of liner 16, it should be appreciated that when threading the elongate member 36 through the base aperture(s) 18A, 18B, a piece of liner 16 will typically be included between the adhesive 14 and the elongate member 36 to prevent exposure of the elongate member 36 to the adhesive 14.

Alternatively, instead of including a liner 16 as an element of the attachment device 10, it is further contemplated that the adhesive 14 that corresponds with the location of the liner 16 may be removed from the base 12 (referring to the liner 16A, 16B, and 16C illustrated in Figures 4A-6B). By removing portions of the adhesive 14 that correspond with the location of the liner 16A, 16B, and 16C illustrated in the figures, the liner 16A, 16B, and 16C may be unnecessary, and therefore, the liner 16A, 16B, and 16C may be omitted from the attachment device 10.

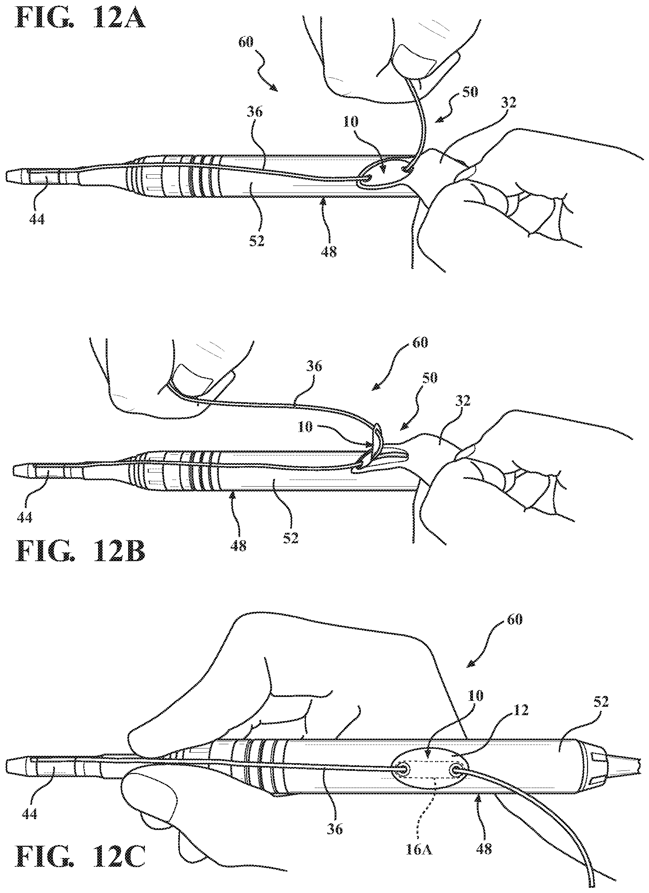

Referring to Figures 12A, 12B, and 12C, an exemplary configuration of a surgical system 60 is illustrated. The surgical system 60 may be configured to comprise a medical instrument 48 and the irrigation assembly 50 described above. The medical instrument 48 may comprise a medical instrument accessory 44, such as a cutting accessory or irrigation sleeve. The elongate member 36 of the irrigation assembly 50 may be threaded through the base aperture(s) 18A, 18B of the attachment device 10, and the distal end 38 coupled to the medical instrument accessory 44.

Referring to Figure 12B, the attachment device 10 may be removed from the indicator tab 32, exposing a portion or area of the adhesive 14 that is not covered by the liner 16. The attachment device 10 may then be removably coupled to the medical instrument 48 via the exposed portion of the adhesive 14. The attachment device 10 may be coupled to the medical instrument 48 at any point along the body 52 of the medical instrument 48, as illustrated in Figure 12C. For example, the attachment device 10 may be coupled to the body 52 of the medical instrument 48 proximate to the medical instrument accessory 44. Alternatively, the attachment device 10 may be coupled to the body 52 of the medical instrument 48 distal to the medical instrument accessory 44. The medical professional may select the location to couple the attachment device 10 to the medical instrument 48. For example, the medical professional may consider the size of elongate member, type of medical procedure to be performed, ergonomic grip of the medical instrument 48, or the like, when selecting the location to couple the attachment device 10 to the medical instrument 48.

As illustrated in Figure 12C, the elongate member 36 is threaded through the base aperture(s) 18A, 18B of the attachment device 10 such that the elongate member 36 is axially aligned with the liner 16A when attached to the body 52 of the medical instrument. A portion of the elongate member 36 is positioned between the liner 16A and the body 52 of the medical instrument 48 when the attachment device 10 is coupled to the medical instrument 48. The presence of the liner 16A between the adhesive 14 and the elongate member 36 allows for the elongate member 36 to be axially slid while attached to the medical instrument 48. This allows for the medical professional to easily adjust the elongate member 36 to be taut, or provide additional slack in the elongate member 36 when needed.

### Method of Securing an Elongate Member to a Medical Instrument:

Furthermore, Figures 12A, 12B, and 12C illustrate various steps of an exemplary method of attaching the elongate member 36 to the medical instrument 48 using the attachment device 10. The method of attaching the elongate member 36 to the medical device may comprise providing the attachment device 10 and or assembly 30, wherein the attachment device 10 comprises the liner 16, the base 12, and the adhesive 14 disposed at least partially between the base 12 and the liner 16. The attachment device 10 may further comprise the first base aperture 18A and the second base aperture 18B. The liner 16 may be positioned adjacent to the adhesive 14, such that the liner 16 is at least partially between the first base aperture 18A and the second base aperture 18B and adjacent to the adhesive. The method may further comprise threading the elongate member 36 through the first base aperture 18A and the second base aperture 18B of the attachment device 10, such that the elongate member 36 is axially aligned with the liner 16. Alternatively, as described above with reference to the liner 16C configuration illustrated in Figures 6A and 6B, the liner 16C may be positioned proximate to the base aperture(s) 18A, 18B and positioned adjacent to the adhesive 14. The liner 16C may be aligned with the longitudinal axis defined by the base apertures 18A, 18B.

The method of securing the elongate member 36 may further comprise adhering the attachment device 10 to the medical instrument 48. The attachment device 10 may be removably coupled to the body 52 of the medical instrument 48 by pressing the exposed portion of the adhesive 14 to the body 52 of the medical instrument 48, such that the elongate member 36 is at least partially disposed between the attachment device 10 and the medical instrument 48. For example, the attachment device 10 including the elongate member 36 threaded through the aperture(s) 18A, 18B may be pressed against the base 52 to secure the attachment device 10 to the medical instrument 48.

The method may further comprise separating the indicator tab 32 of the attachment device assembly 30 described above from the adhesive 14. The indicator tab 32 may be separated or removed by pulling on the elongate member 36 that is threaded through the attachment device 10 of the assembly 30. The indicator tab 32 may be configured to comprise indicia 34 representative of the method of coupling the attachment device 10 to the medical instrument 48.

The method may further comprise axially sliding the elongate member 36 adjacent to the liner 16 such that the elongate member 36 is taut when the attachment device 10 is coupled to the medical instrument 48. For example, pulling on the proximal end 40 of the elongate member 36 to remove any slack in the distal end 38 of the elongate member 36. This may reduce the profile and or bulkiness of the surgical system 60. It may also prevent the elongate member 36 from becoming caught on or entangled with something during operation of the medical instrument 48. Alternatively, the elongate member 36 may be slid by pulling on the distal end 38 to create additional slack in the elongate member 36.

The method may also comprise removing the second liner 22 from the adhesive 14, wherein removal of the second liner 22 exposes the portion of the adhesive 14 configured to removably couple the attachment device 10 to the medical instrument 48.

After adhering the attachment device 10 to the medical instrument 48, the method may comprise separating the attachment device 10 from the medical instrument 48 by pulling on the elongate member 36. For example, pulling on the proximal end 40 of the elongate member 36 may remove and/or detach the attachment device 10 from the medical instrument 48. Allowing the elongate member and attachment device 10 to be disposed of following a medical procedure as part of the sterilization process.

### Method of Manufacture

A method of manufacturing the attachment device 10 and/or assembly 30 that is described above may comprise providing a base 12 having a top surface and an opposing bottom surface. One or more base aperture(s) 18A, 18B may be cut in the base 12. For example, a die cutting process may be utilized to cut the first base apertures 18A and the second base aperture 18B. The adhesive 14 may be applied to the bottom surface of the base 12. The adhesive 14 may be applied to the base 12 such that the adhesive 14 is flush to the first base aperture18A and the second base aperture 18B. Alternatively, the adhesive 14 may be applied to the base 12 such that a portion of the base 12 surrounding the first base aperture 18A and the second base aperture 18B is free from the adhesive 14.

The method may further comprise removably coupling the liner 16A, 16B, 16C to the adhesive 14, such that the liner 16A, 16B is at least partially disposed between the first base aperture 18A and the second base aperture 18B in the base 12. Alternatively, as described above, the liner 16C may be configured such that it is not positioned between the first base aperture 18A and the second base aperture 18B.

The method may also comprise cutting a recess 20, bevel, chamfer, fillet, or similar transition in the top surface of the base 12 configured to surround each of the first base aperture 18A and the second base aperture 18B. The recess 20 may be created by making a secondary cut in any number of shapes, such as a circular cut. The shape of the recess 20 created by the secondary cut may correspond to the shape of the first base aperture 18A and the second base aperture 18B. Alternatively, the shape of the recess 20 may be different from the shape of the first base aperture 18A and the second base aperture 18B. Generally, the size and/or dimensions of the recess 20 created by the secondary cut may be larger than the size and/or dimensions of the first base aperture 18A and the second base aperture 18B. For example, wherein the secondary cut is a circular cut, the secondary cut may be centered at the midpoint of the first base aperture 18A and the second base aperture 18B, wherein the diameter of the secondary cut to create the recess 20 is larger than the diameter of the first base aperture 18A and the second base aperture 18B. The secondary cut configured to create the recess may also be referred to as a "kiss-cut" or a relief cut.

It is further contemplated that the attachment device 10 may be manufactured by starting pre-assembled piece of material that may include the base 12, the adhesive 14 applied to one surface of the base 12, and the liner 16A, 16B, 16C removably coupled to the adhesive 14. In this configuration, the method of manufacturing the attachment device 10 may comprise cutting one or more base aperture(s) 18A, 18B through the base 12, the adhesive 14, and the liner16A, 16B, 16C. The method may further comprise cutting the liner 16A, 16B, 16C such that the liner 16A, 16B is at least partially disposed between the first base aperture 18A and the second base aperture 18B in the base 12. Alternatively, as described above, the liner 16A, 16B, 16C may be cut such that the liner 16C it is not positioned between the first base aperture 18A and the second base aperture 18B. The method may also comprise cutting a recess 20, bevel, chamfer, fillet, or similar transition in the top surface of the base 12 configured to surround each of the first base aperture 18A and the second base aperture 18B. The recess 20 may be created by making a secondary cut in any number of shapes, such as a circular cut. Similar to as described above, the size and/or dimensions of the recess 20 created by the secondary cut may be larger than the size and/or dimensions of the first base aperture 18A and the second base aperture 18B. The secondary cut configured to create the recess may also be referred to as a "kiss-cut" or a relief cut.

The method may further comprise threading the elongate member 36 through the first base aperture 18A and the second base aperture 18B. Utilizing the liner 16A, 16B configuration illustrated in Figures 4A-5B, the elongate member 36 may be threaded with the attachment device 10 by starting the elongate member 36 adjacent to the top surface of the base 12, inserting the elongate member 36 through the first base aperture 18A, extending the elongate member 36 along the liner 16A, 16B, and inserting the elongate member 36 through the second base aperture 18B. Alternatively, utilizing the liner 16C configuration illustrated in Figures 6A and 6B, the elongate member 36 may be threaded with the attachment device 10 by starting the elongate member 36 adjacent to the liner 16C, inserting the elongate member 36 through the first base aperture 18A, extending the elongate member 36 along the top surface of the base 12, and inserting the elongate member through the second base aperture 18B.

The method may also comprise removably coupling an indicator tab 32 to the adhesive 14 proximate to the liner 16, wherein the indicator tab 32 may be configured to comprise indicia 34 representative of said method of coupling the attachment device to the medical instrument. The indicia 34 may include text, graphics, and/or images representing the method of coupling the elongate member 36 to the medical instrument. The indicia 34 may be printed on the indicator tab 32 as part of the method of manufacturing the attachment device 10 and/or assembly 30. For example, referring back to Figure 9, an example of the indicia 34 that may be printed on the indicator tab 32 is illustrated. The indicia 32 include graphics and/or numerals indicating the steps of attaching the elongate member 36 to the cutting accessory, securing the elongate member 36 to the medical instrument 48 using the attachment device 10, and connecting the elongate member 36 to the medical system, such as an irrigation system.

As described above, the liner 16 may comprise a second liner 22, wherein the second liner 22 is integrally formed with the indicator tab 32. In manufacturing the attachment device assembly 30, the liner 16 and second liner 22 may initially comprise a single integral piece of liner 16, 22. However, as the liner 16 is configured to remain coupled to the adhesive 14 when the indicator tab 32 is removed, it may be necessary to cut the liner 16, 22 to allow for easy separation of the liner 16 and second liner 22 when the attachment device 10 is removed from the indicator tab 32. Therefore, the method of manufacturing the attachment device 10 may further comprise cutting the liner 16, 22 such that the second liner 22 may be configured to be separable from the liner 16, and wherein the liner 16 remains at least partially disposed between the first base aperture 18A and the second base aperture 18B.

The method may also comprise cutting the liner aperture(s) 21A, 21B in the liner 16A, wherein the liner aperture(s) 21A, 21B is aligned with the base aperture(s) 18A, 18B. The base aperture(s) 18A, 18B may be defined by a first diameter, and the liner aperture(s) 21A may be defined by a second diameter. The attachment device 10 may be configured such that the first and second diameter are equal. However, it is also contemplated that the second diameter may be larger than the first diameter, and/or the first diameter may be larger than the second diameter.

Several embodiments have been discussed in the foregoing description. However, the embodiments discussed herein are not intended to be exhaustive or limit the attachment device 10, attachment device assembly 30, and/or the surgical system 60 to any particular form. The terminology which has been used is intended to be in the nature of words of description rather than of limitation. Many modifications and variations are possible in light of the above teachings.

## Claims

1. An attachment device (10) for securing an elongate member (36) to a medical instrument (48), said attachment device (10) comprising:
a liner (16; 22);
a base (12) comprising a first base aperture (18A) and a second base aperture (18B);
an adhesive (14);
wherein said adhesive (14) is disposed at least partially between said base (12) and said liner (16; 22); and
wherein said liner (16; 22) is at least one of
(i) at least partially between said first base aperture (18A) and said second base aperture (18B) and is adjacent to said adhesive (14), and
(ii) configured to cover a portion of the adhesive (14) disposed on the base (12) between the first base aperture (18A) and a first outer edge of the attachment device (10), and between the second base aperture (18B) and a second outer edge of the attachment device (10);
**characterized in that**
said liner (16; 22) is configured to allow the elongate member (36) to move axially along an axis (L) defined by said first base aperture (18A) and said second base aperture (18B) when said attachment device (10) is attached to the medical instrument (48) via an exposed portion of the adhesive (14).

2. The attachment device (10) of claim 1,
wherein said base (12) further comprises a recess (20) surrounding said first base aperture (18A) and said second base aperture (18B), and/or
wherein said attachment device (10) further comprises indicia representative of a method of attaching said attachment device (10) to the medical instrument (48).

3. The attachment device (10) of any one of claims 1 and 2, wherein said liner (16; 22) further comprises a first liner portion (16) and a second liner portion (22);
wherein said first liner portion (16) is disposed at least partially between said first base aperture (18A) and said second base aperture (18B);
wherein said second liner portion (22) is separable from said first liner portion (16); and
wherein said first liner portion (16) is configured to remain coupled to said adhesive (14) when said second liner portion (22) is removed.

4. The attachment device (10) of any one of claims 1-3, wherein said liner (16) further comprises a first liner aperture (21A) and a second liner aperture (21B);
wherein said first liner aperture (21A) is aligned with said first base aperture (18A); and
wherein said second liner aperture (21B) is aligned with said second base aperture (18B).

5. The attachment device (10) of claim 4, wherein said first base aperture (18A) is defined by a first diameter, and said first liner aperture (21A) is defined by a second diameter; and
wherein said second diameter is larger than said first diameter.

6. An attachment device assembly (30) for securing an elongate member (36) to a medical instrument (48), said assembly (30) comprising:
the attachment device (10) of claim 1 or of claim 3 as far as dependent on claim 1; and
an indicator tab (32) removably coupled to said attachment device (10), said indicator tab (32) comprising indicia representative of a method of attaching said attachment device (10) to the medical instrument (48).

7. The attachment device assembly (30) of claim 6, wherein said second liner portion (22) is integrally formed with said indicator tab (32).

8. An attachment device assembly (30) for use with a medical instrument (48) to facilitate irrigation, said assembly (30) comprising:
the attachment device (10) of claim 1; and
the elongate member (36), with the elongate member (36) threaded through said first base aperture (18A) and said second base aperture (18B), and adapted to provide irrigation.

9. The attachment device assembly (30) of claim 8, wherein said liner (16; 22) is removably coupled to said adhesive (14) such that said adhesive (14) is at least partially disposed between said base (12) and said liner (16; 22).

10. The attachment device assembly (30) of any one of claims 8 and 9,
wherein said elongate member (36) further comprises a first end and an opposing second end;
wherein said first end comprises a first coupler configured to couple said elongate member (36) to the medical instrument (48); and
wherein said second end comprises a second coupler configured to couple said elongate member (36) to a fluid source.

11. The attachment device assembly (30) of claim 9,
wherein said liner (16; 22) is configured to align with said elongate member (36) when said base (12) is coupled to the medical instrument (48) by said adhesive (14), and
wherein, optionally, the attachment device assembly (30) further comprises an indicator tab (32) removably coupled to a section of said adhesive (14) proximate to said liner (16; 22), said indicator tab (32) comprising indicia representative of a method of attaching said attachment device (10) to the medical instrument (48).

12. An irrigation system comprising:
a medical instrument (48) configured to cut tissue, said medical instrument (48) having a body configured to be held by a user; and
the attachment device assembly (30) of claim 8,
wherein a coupling of said elongate member (36) to said medical instrument (48) by the attachment device (10) of the attachment assembly (30) is removable; and
wherein said elongate member (36) is threaded through said first base aperture (18A) and said second base aperture (18B) such that a portion of said elongate member (36) is adjacent to said body of said medical instrument (48).

13. The irrigation system of claim 12, wherein said attachment device (10) further comprises an indicator tab (32) removably coupled to a section of said adhesive (14) proximate to said liner (16; 22), said indicator tab (32) configured to comprise indicia representative of a method of attaching said attachment device (10) to said medical instrument (48).

14. A method of coupling an elongate member (36) to a medical instrument (48), said method comprising:
providing an attachment device (10) according to claim 1;
threading an elongate member (36) through the first base aperture (18A) and the second base aperture (18B) such that the elongate member (36) is axially aligned with the liner (16; 22); and
adhering the attachment device (10) to the medical instrument (48), wherein an exposed portion of the adhesive (14) removably couples the attachment device (10) to the medical instrument (48) such that the elongate member (36) is at least partially disposed between the attachment device (10) and the medical instrument (48).

15. The method of claim 14,
wherein said attachment device (10) further comprises an indicator tab (32) removably coupled to a section of said adhesive (14) proximate to said liner (16; 22), said indicator tab (32) configured to comprise indicia representative of the method of coupling the elongate member (36) to said medical instrument (48), and said method further comprises:
separating the indicator tab (32) from the adhesive (14) by pulling on the elongate member(36); and/or
wherein said method further comprises:
removing a second portion (22) of the liner (16; 22) from the adhesive (14), wherein removal of the second portion (22) of the liner (16; 22) exposes the exposed portion of the adhesive (14) configured to removably couple the attachment device (10) to the medical instrument (48).

## Patentansprüche

1. Befestigungsvorrichtung (10) zum Sichern eines länglichen Elements (36) an einem medizinischen Instrument (48), wobei die Befestigungsvorrichtung (10) umfasst:
einen Liner (16; 22);
eine Basis (12), die eine erste Basisöffnung (18A) und eine zweite Basisöffnung (18B) umfasst;
ein Klebemittel (14);
wobei das Klebemittel (14) zumindest zum Teil zwischen der Basis (12) und dem Liner (16; 22) vorgesehen ist; und
wobei der Liner (16; 22)
(i) sich zumindest zum Teil zwischen der ersten Basisöffnung (18A) und der zweiten Basisöffnung (18b) und benachbart zu dem Klebemittel (14) befindet, und/oder
(ii) dazu ausgebildet ist, einen Teil des auf der Basis (12) vorgesehenen Klebemittels (14) zwischen der ersten Basisöffnung (18A) und einer ersten Außenkante der Befestigungsvorrichtung (10) sowie zwischen der zweiten Basisöffnung (18B) und einer zweiten Außenkante der Befestigungsvorrichtung (10) abzudecken;
**dadurch gekennzeichnet, dass**
der Liner (16; 22) dazu ausgebildet ist, eine axiale Bewegung des länglichen Elements (36) entlang einer durch die erste Basisöffnung (18A) und die zweite Basisöffnung (18B) definierte Achse (L) zu ermöglichen, wenn die Befestigungsvorrichtung (10) mittels eines freiliegenden Teils des Klebemittels (14) an dem medizinischen Instrument (48) befestigt ist.

2. Befestigungsvorrichtung (10) nach Anspruch 1,
wobei die Basis (12) ferner eine die erste Basisöffnung (18A) und die zweite Basisöffnung (18B) umgebende Aussparung (20) umfasst, und/oder
wobei die Befestigungsvorrichtung (10) ferner Angaben umfasst, die ein Verfahren zum Befestigen der Befestigungsvorrichtung (10) an dem medizinischen Instrument (48) repräsentieren.

3. Befestigungsvorrichtung (10) nach einem der Ansprüche 1 und 2, wobei der Liner (16; 22) ferner einen ersten Linerteil (16) und einen zweiten Linerteil (22) umfasst;
wobei der erste Linerteil (16) zumindest zum Teil zwischen der ersten Basisöffnung (18A) und der zweiten Basisöffnung (18B) vorgesehen ist;
wobei der zweite Linerteil (22) von dem ersten Linerteil (16) trennbar ist; und
wobei der erste Linerteil (16) dazu ausgebildet ist, mit dem Klebemittel (14) gekoppelt zu bleiben, wenn der zweite Linerteil (22) entfernt ist.

4. Befestigungsvorrichtung (10) nach einem der Ansprüche 1-3, wobei der Liner (16) ferner eine erste Lineröffnung (21A) und eine zweite Lineröffnung (21B) umfasst;
wobei die erste Lineröffnung (21A) mit der ersten Basisöffnung (18A) ausgerichtet ist; und
wobei die zweite Lineröffnung (21B) mit der zweiten Basisöffnung (18B) ausgerichtet ist.

5. Befestigungsvorrichtung (10) nach Anspruch 4, wobei die erste Basisöffnung (18A) durch einen ersten Durchmesser definiert ist und die erste Lineröffnung (21A) durch einen zweiten Durchmesser definiert ist; und
wobei der zweite Durchmesser größer ist als der erste Durchmesser.

6. Befestigungsvorrichtungsanordnung (30) zum Sichern eines länglichen Elements (36) an einem medizinischen Instrument (48), wobei die Anordnung (30) umfasst:
die Befestigungsvorrichtung (10) nach Anspruch 1 oder nach Anspruch 3, sofern abhängig von Anspruch 1; und
einen Anzeigetab (32), der entfernbar mit der Befestigungsvorrichtung (10) gekoppelt ist, wobei der Anzeigetab (32) Angaben umfasst, die ein Verfahren zum Befestigen der Befestigungsvorrichtung (10) an dem medizinischen Instrument (48) repräsentieren.

7. Befestigungsvorrichtungsanordnung (30) nach Anspruch 6, wobei der zweite Linerteil (22) mit dem Anzeigetab (32) integral ausgebildet ist.

8. Befestigungsvorrichtungsanordnung (30) zur Verwendung mit einem medizinischen Instrument (48), um eine Irrigation zu ermöglichen, wobei die Anordnung (30) umfasst:
die Befestigungsvorrichtung (10) nach Anspruch 1; und
das längliche Element (36), wobei das längliche Element (36) durch die erste Basisöffnung (18A) und die zweite Basisöffnung (18B) eingefädelt und dazu ausgebildet ist, Irrigation bereitzustellen.

9. Befestigungsvorrichtungsanordnung (30) nach Anspruch 8, wobei der Liner (16; 22) derart lösbar mit dem Klebemittel (14) gekoppelt ist, dass das Klebemittel (14) zumindest zum Teil zwischen der Basis (12) und dem Liner (16; 22) vorgesehen ist.

10. Befestigungsvorrichtungsanordnung (30) nach einem der Ansprüche 8 und 9,
wobei das längliche Element (36) ferner ein erstes Ende und ein gegenüberliegendes zweites Ende umfasst;
wobei das erste Ende einen ersten Koppler umfasst, der dazu ausgebildet ist, das längliche Element (36) mit dem medizinischen Instrument (48) zu verbinden; und
wobei das zweite Ende einen zweiten Koppler umfasst, der dazu ausgebildet ist, das längliche Element (36) mit einer Fluidquelle zu verbinden.

11. Befestigungsvorrichtungsanordnung (30) nach Anspruch 9,
wobei der Liner (16; 22) dazu ausgebildet ist, sich mit dem länglichen Element (36) auszurichten, wenn die Basis (12) mittels des Klebemittels (14) mit dem medizinischen Instrument (48) gekoppelt ist, und
wobei die Befestigungsvorrichtungsanordnung (30) optional ferner einen Anzeigetab (32) umfasst, der entfernbar mit einem unmittelbar an den Liner (16; 22) anschließenden Abschnitt des Klebemittels (14) gekoppelt ist, wobei der Anzeigetab (32) Angaben umfasst, die ein Verfahren zum Befestigen der Befestigungsvorrichtung (10) an dem medizinischen Instrument (48) repräsentieren.

12. Irrigationssystem umfassend:
ein medizinisches Instrument (48), das dazu ausgebildet ist, Gewebe zu schneiden, wobei das medizinische Instrument (48) einen Körper hat, der dazu ausgebildet ist, von einem Benutzer gehalten zu werden; und
die Befestigungsvorrichtungsanordnung (30) nach Anspruch 8,
wobei eine Kopplung des länglichen Elements (36) mit dem medizinischen Instrument (48) mittels der Befestigungsvorrichtung (10) der Befestigungsvorrichtungsanordnung (30) entfernbar ist; und
wobei das längliche Element (36) durch die erste Basisöffnung (18A) und die zweite Basisöffnung (18B) derart durchgefädelt ist, dass sich ein Teil des länglichen Elements (36) benachbart zu dem Körper des medizinischen Instruments (48) befindet.

13. Irrigationssystem nach Anspruch 12, wobei die Befestigungsvorrichtung (10) ferner einen Anzeigetab (32) umfasst, der entfernbar mit einem unmittelbar an den Liner (16; 22) anschließenden Abschnitt des Klebemittels (14) gekoppelt ist, wobei der Anzeigetab (32) dazu ausgebildet ist, Angaben zu umfassen, die ein Verfahren zum Befestigen der Befestigungsvorrichtung (10) an dem medizinischen Instrument (48) repräsentieren.

14. Verfahren zum Koppeln eines länglichen Elements (36) mit einem medizinischen Instrument (48), umfassend:
Bereitstellen einer Befestigungsvorrichtung (10) nach Anspruch 1;
Einfädeln eines länglichen Elements (36) durch die erste Basisöffnung (18A) und die zweite Basisöffnung (18B) derart, dass das längliche Element (36) axial mit dem Liner (16; 22) ausgerichtet ist; und
Ankleben der Befestigungsvorrichtung (10) an dem medizinischen Instrument (48), wobei ein freiliegender Teil des Klebemittels (14) die Befestigungsvorrichtung (10) derart entfernbar mit dem medizinischen Instrument (48) koppelt, dass das längliche Element (36) zumindest zum Teil zwischen der Befestigungsvorrichtung (10) und dem medizinischen Instrument (48) vorgesehen ist.

15. Verfahren nach Anspruch 14,
wobei die Befestigungsvorrichtung (10) ferner einen Anzeigetab (32) umfasst, der entfernbar mit einem unmittelbar an den Liner (16; 22) anschließenden Abschnitt des Klebemittels (14) gekoppelt ist, der Anzeigetab (32) dazu ausgebildet ist, Angaben zu umfassen, die das Verfahren zum Koppeln des länglichen Elements (36) mit dem medizinischen Instrument (48) repräsentieren, und das Verfahren ferner umfasst:
Trennen des Anzeigetabs (32) von dem Klebemittel (14) durch Ziehen an dem länglichen Element (36); und/oder
wobei das Verfahren ferner umfasst:
Entfernen eines zweiten Teils (22) des Liners (16; 22) von dem Klebemittel (14), wobei das Entfernen des zweiten Teils (22) des Liners (16; 22) den freiliegenden Teil des Klebemittels (14) freilegt, der dazu ausgebildet ist, die Befestigungsvorrichtung (10) entfernbar mit dem medizinischen Instrument (48) zu koppeln.

## Revendications

1. Dispositif de fixation (10) permettant de fixer un élément allongé (36) à un instrument médical (48), ledit dispositif de fixation (10) comprenant:
un revêtement (16; 22);
une base (12) comprenant une première ouverture de base (18A) et une seconde ouverture de base (18B);
un adhésif (14);
dans lequel ledit adhésif (14) est disposé au moins en partie entre ladite base (12) et ledit revêtement (16; 22); et
dans lequel ledit revêtement (16; 22) est au moins
(i) disposée au moins en partie entre ladite première ouverture de base (18A) et ladite seconde ouverture de base (18B) et est adjacente audit adhésif (14), et
(ii) conçue pour recouvrir une partie de l'adhésif (14) disposé sur la base (12) entre la première ouverture de base (18A) et un premier bord extérieur du dispositif de fixation (10), et entre la seconde ouverture de base (18B) et un second bord extérieur du dispositif de fixation (10);
**caractérisé en ce que**
ledit revêtement (16; 22) est conçu pour permettre à l'élément allongé (36) de se déplacer axialement le long d'un axe (L) défini par ladite première ouverture de base (18A) et ladite seconde ouverture de base (18B) lorsque le dispositif de fixation (10) est fixé à l'instrument médical (48) par l'intermédiaire d'une partie apparente de l'adhésif (14).

2. Dispositif de fixation (10) selon la revendication 1,
dans lequel ladite base (12) comprend en outre un évidemment (20) entourant ladite première ouverture de base (18A) et ladite seconde ouverture de base (18B), et/ou
dans lequel ledit dispositif de fixation (10) comprend en outre des indications montrant un procédé de fixation dudit dispositif de fixation (10) à l'instrument médical (48).

3. Dispositif de fixation (10) selon l'une quelconque des revendications 1 et 2, dans lequel ledit revêtement (16; 22) comprend en outre une première partie de revêtement (16) et une seconde partie de revêtement (22);
dans lequel ladite première partie de revêtement (16) est disposée au moins en partie entre ladite première ouverture de base (18A) et ladite seconde ouverture de base (18B);
dans lequel ladite seconde partie de revêtement (22) peut être séparée de ladite première partie de revêtement (16); et
dans lequel ladite première partie de revêtement (16) est conçue pour rester accouplée audit adhésif (14) lorsque ladite seconde partie de revêtement (22) est retirée.

4. Dispositif de fixation (10) selon l'une quelconque des revendications 1 à 3, dans lequel ledit revêtement (16) comprend en outre une première ouverture de revêtement (21A) et une seconde ouverture de revêtement (21B);
dans lequel ladite première ouverture de revêtement (21A) est alignée avec ladite première ouverture de base (18A); et
dans lequel ladite seconde ouverture de revêtement (21A) est alignée avec ladite seconde ouverture de base (18A).

5. Dispositif de fixation (10) selon la revendication 1, dans lequel ladite première ouverture de base (18A) est définie par un premier diamètre et ladite première ouverture de ligne (21A) est définie par un second diamètre; et
dans lequel ledit second diamètre est supérieur audit premier diamètre.

6. Ensemble dispositif de fixation (30) permettant de fixer un élément allongé (36) à un instrument médical (48), ledit ensemble dispositif de fixation (30) comprenant:
le dispositif de fixation (10) selon la revendication 1 ou selon la revendication 3 tant qu'elle est dépendante de la revendication 1; et
une languette indicatrice (32) accouplée amovible audit dispositif de fixation (10), ladite languette indicatrice (32) comprenant des indices représentant un procédé de fixation dudit dispositif de fixation (10) à l'instrument médical (48).

7. Ensemble dispositif de fixation (30) selon la revendication 6, dans lequel ladite seconde partie de revêtement (22) est formé d'une seule pièce avec ladite languette indicatrice (32).

8. Ensemble dispositif de fixation (30) destiné à être utilisé avec un instrument médical (48) afin de faciliter l'irrigation, ledit ensemble (30) comprenant:
le dispositif de fixation (10) selon la revendication 1; et
l'élément allongé (36), l'élément allongé (36) étant vissé dans ladite première ouverture de base (18A) et dans ladite seconde ouverture de base (18B), et conçu pour assurer l'irrigation.

9. Ensemble dispositif de fixation (30) selon la revendication 8, dans lequel ledit revêtement (16; 22) est accouplé amovible audit adhésif (14) de sorte que ledit adhésif (14) soit au moins en partie disposé entre ladite base (12) et ledit revêtement (16; 22).

10. Ensemble dispositif de fixation (30) selon l'une quelconque des revendications 8 et 9,
dans lequel ledit élément allongé (36) comprend en outre une première extrémité et une seconde extrémité en regard;
dans lequel ladite première extrémité comprend un premier dispositif d'accouplement pour accoupler ledit élément allongé (36) à l'instrument médical (8); et
dans lequel ladite seconde extrémité comprend un second dispositif d'accouplement pour accoupler ledit élément allongé (36) à une source de fluide.

11. Ensemble dispositif de fixation (30) selon la revendication 9,
dans lequel ledit revêtement (16; 22) est conçu pour s'aligner avec ledit élément allongé (36) lorsque ladite base (12) est accouplée à l'instrument médical (48) par ledit adhésif (14), et
dans lequel, éventuellement, l'ensemble dispositif de fixation (30) comprend en outre une languette indicatrice (32) accouplée amovible à une section dudit adhésif (14) à proximité de ledit revêtement (16; 22), ladite languette indicatrice (32) comprenant des indications représentant un procédé de fixation dudit dispositif de fixation (10) à l'instrument médical (48).

12. Système d'irrigation comprenant:
un instrument médical (48) conçu pour couper les tissus, ledit instrument médical (48) ayant un corps conçu pour être maintenu par un utilisateur; et
l'ensemble dispositif de fixation (30) selon la revendication 8,
dans lequel un accouplement dudit élément allongé (36) audit instrument médical (48) par le dispositif de fixation (10) de l'ensemble de fixation (30) peut être retiré; et
dans lequel ledit élément allongé (36) est vissé dans ladite première ouverture de base (18A) et dans ladite seconde ouverture de base (18B) de sorte qu'une partie dudit élément allongé (36) soit adjacente audit corps dudit instrument médical (48).

13. Système d'irrigation selon la revendication 12, dans lequel, ledit dispositif de fixation (10) comprend en outre une languette indicatrice (32) accouplée amovible à une section dudit adhésif (14) à proximité de ledit revêtement (16; 22), ladite languette indicatrice (32) est conçue pour comprendre des indices représentant un procédé de fixation dudit dispositif de fixation (10) à l'instrument médical (48).

14. Procédé d'accouplement d'un élément allongé (36) à un instrument médical (48), ledit procédé consistant à:
utiliser un dispositif de fixation (10) selon la revendication 1 ;
visser un élément allongé (36) dans la première ouverture de base (18A) et dans la seconde ouverture de base (18B) de sorte que l'élément allongé (36) est aligné axialement avec le revêtement (16; 22); et
faire adhérer le dispositif de fixation (10) à l'instrument médical (48), dans lequel une partie apparente de l'adhésif (14) accouple amovible le dispositif de fixation (10) à l'instrument médical (48) de sorte que l'élément allongé (36) soit au moins en partie disposé entre le dispositif de fixation (10) et l'instrument médical (48).

15. Procédé selon la revendication 14,
dans lequel ledit dispositif de fixation (10) comprend en outre une languette indicatrice (32) accouplée amovible à une section dudit adhésif (14) à proximité de ledit revêtement (16; 22), ladite languette indicatrice (32) étant conçue pour comprendre des repères représentant un procédé d'accouplement de l'élément allongé (36) audit instrument médical (48), et ledit procédé consiste en outre à:
séparer la languette indicatrice (32) de l'adhésif (14) en tirant sur l'élément allongé (36); et/ou
le procédé consistant en outre à :
retirer une seconde partie (22) du revêtement (16; 22) de l'adhésif (14), dans lequel le retrait de la seconde partie (22) du revêtement (16; 22) rend visible la partie apparente de l'adhésif (14) conçu pour accoupler de manière amovible le dispositif de fixation (10) à l'instrument médical (48).
